# EUROPEAN PATENT SPECIFICATION

(11) **EP 4 314 727 B1**
(45) Date of publication and mention of the grant of the patent: **01.07.2026**
(21) Application number: 22710824.8
(22) Date of filing: 24.02.2022
(51) Int. Cl.: G01F 23/24, G01F 23/26, G01F 23/263, G01F 23/292, G01F 23/296, F24F 6/00, B65D 85/00, C12M 1/34, B65D 90/00, C12M 1/00, C12M 3/00

(54) **FILL LEVEL DETECTION**
FÜLLSTANDSDETEKTION
DÉTECTION DE NIVEAU DE REMPLISSAGE

(30) Priority: 25.03.2021 US 202163165842 P
(43) Date of publication of application: 07.02.2024
(73) Proprietor: Nexpring US Opco Inc., Beverly, MA 01915 (US)
(72) Inventor: CHAND, Tony, Brisbane, QLD 4113 (AU); FORD, Michael, Brisbane, QLD 4113 (AU); SHRESTHA, Ravi, Maple Grove, MN 53311 (US); SYLVAIN, Jonathan, Lakewood, CO 80227 (US)
(74) Representative: Leach, Sean Adam
(86) International application number: PCT/US2022/017666
(87) International publication number: WO 2022/203800

(56) References cited:
- US-A1- 2011 314 907
- US-A1- 2012 122 201
- US-A1- 2016 041 021
- US-A1- 2019 041 075
- US-A1- 2021 041 283

## Description

### Technical Field

This disclosure relates to a system for fill level detection in a flask.

### Background

In various systems, the operation of a device may be dependent on the fill level of liquid in a flask. In an example, the fill level of liquid in a fuel tank may be indicative of a remaining duration of operation for vehicle. In another example, the fill level of flask in a humidification system may be indicative of the remaining duration of operation of the humidification system. In various scenarios, cessation of proper operation of devices using flasks for hosting liquids may result in catastrophic failure such as the stranding of passengers, sample dry out, or other failures. Hence, improvements in the timing of signaling for ameliorative responses to avoid failures due to low (or otherwise improper) fill levels will continue to improve device operations and user experiences. Document US2021041283A1 discloses an incubator.

### SUMMARY

The invention is defined by the subject-matter of the independent claims. The dependent claims are directed to advantageous embodiments.

### BRIEF DESCRIPTION OF THE DRAWINGS

Figure 1 shows an example fill level detection device.
Figure 2 shows a first example of fill-level detection logic is shown
Figure 3 shows second example fill-level detection logic.
Figure 4 shows an example capacitive fill-level detection device.
Figure 5 shows an example gas humidification system.

### DETAILED DESCRIPTION

In various scenarios, a flask (such as a reservoir, tank, or other fluid container) may host a liquid within a device. Hosted liquid may be used in the operation of the device. In some cases, the liquid may be used during proper execution of one or more operational modes of the device. Moreover, operation of the device may deplete the liquid in the device, and/or the liquid may be depleted via processes such as evaporation, leaking, overflow, and/or other processes. Thus, the flask may be monitored to ensure the liquid is present at a fill level consistent with proper operation of the device.

In various scenarios, the flask may be opaque or have its contents otherwise obscured from view. In various scenarios, automated monitoring may be used, e.g., to ensure consistent operation. Accordingly, the flask may include liquid sensor systems to support monitoring. Liquid sensor may include capacitive sensors, optical (e.g., photoelectric) sensors, ionization sensors, and/or other sensors using transmissions (in some cases, including static fields) that extend into the flask over one or more paths between a transmitter/receiver pair. In an illustrative example, a liquid sensor may include electrodes forming a capacitor. The field in the example capacitor may extend into the flask and be affected by the liquid content therein. The capacitance between the electrodes may be used to determine the presence and/or quantity of liquid at a position within the flask.

In various scenarios, the liquid may undergo turbulence. For example, in various gas humidification applications, a gas may be forced to bubble through the liquid in the flask causing turbulence that may prevent a defined fill line from forming at the fill level of the liquid in the flask. Thus, the fill level detection scheme, e.g. used by logic executed by control circuitry within a device, may account an indefinite (or non-existent) fill line.

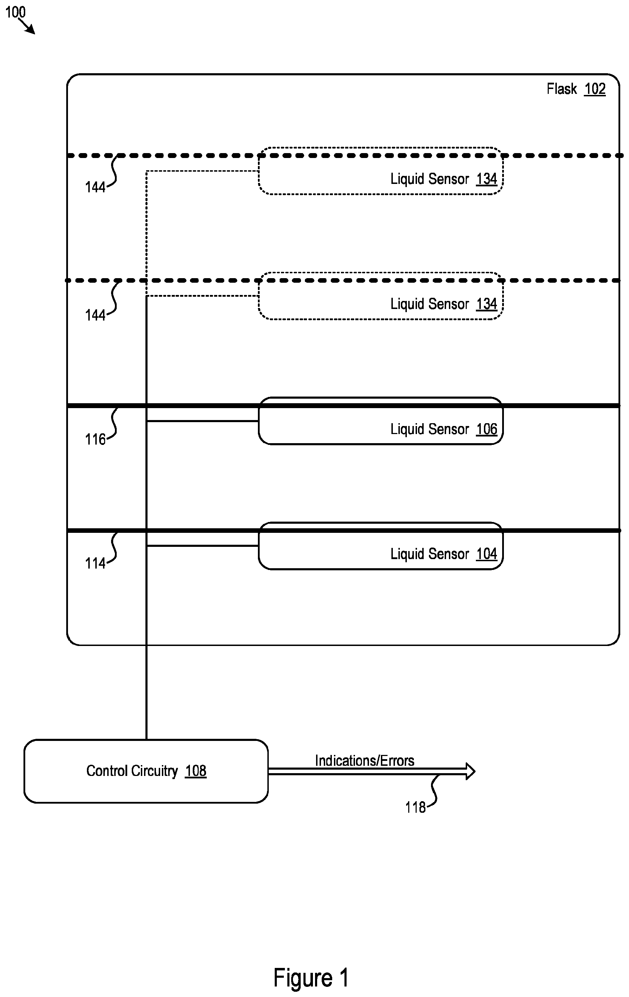

Figure 1 shows an example fill level detection device 100. The device 100 may include a flask 102. The device 100 may include (e.g., two or more) liquid sensors 104, 106, 134 that correspond to fill levels 114, 116, 144 within the flask. The device may include control circuitry 108 that may be in communication with the liquid sensors 104, 106, 134. Using logic (e.g., example logic 200, 300), the control circuitry may generate outputs 118 (e.g., based on measured readings from the liquid sensors 104, 106, 134). The outputs may include such as indications of the fill level of the liquid within the flask 102 and/or errors such as empty flask errors, overfill errors, or other fill-level errors.

First 104 and second 106 liquid sensors may correspond to bottom and elevated sensors. A bottom sensor may correspond to a liquid sensor near or at the bottom of the flask that, for example, may correspond to a lowest fill-level for which a fill-level indication may be generated. An elevated sensor may include a sensor for which there are one or more sensors positioned below the elevated sensor. As shown in the example device 100, the second liquid sensor may correspond to a second-level fill sensor for which there is one sensor positioned below. In various implementations, bottom sensors and sensors at fill level adjacent to or otherwise near the bottom fill level may, for example, be used in the detection of empty flask conditions. Although not shown, in various implementations, first 104 and second 106 liquid sensors may include sensors located near the top of the flask and, for example, may be used in the detection of overfilled flask conditions

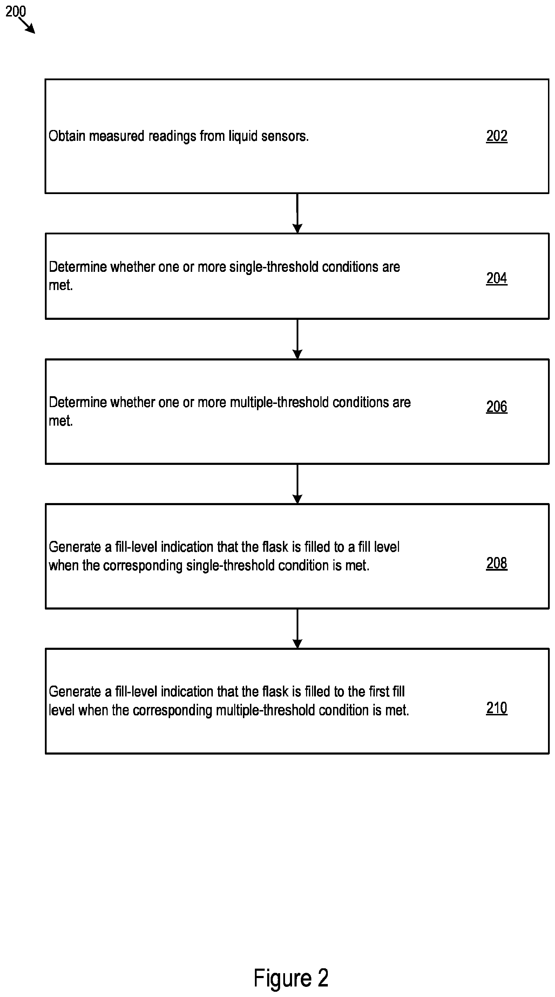

Referring now to Figure 2 while continuing to refer to Figure 1, a first example of fill-level detection logic FLDL 200 is shown.

The FLDL 200 may obtain measured readings from the liquid sensors 104, 106, 134 (202). Obtaining a measured reading may include receiving signal such a raw reading (e.g., an unaltered reading) from the sensor. In some cases, obtaining the measured reading may include adjusting the raw reading to correct for one or more factors.

For example, a measured reading may include an adjustment where a calibrated value (such as value from a raw reading captured while the flask is empty) may be subtracted from the current raw value (e.g., captured during normal operation).

In an example, a measured reading may include an adjustment for temperature. For example, the adjustment may be taken from a lookup table indexed for temperature and/or calculated using an algorithm that uses temperature as an input.

In various implementations, the FLDL 200 may store the measured readings in memory located on the control circuitry for later threshold comparison.

In various implementations, the FLDL 200 may poll the liquid sensors 104, 106, 134 at an interval. For example, the FLDL may poll the sensors periodically (e.g., every minute, every 20 seconds, every 10 seconds, every second) and/or at predetermined intervals.

After obtaining the measured reading from the second liquid sensor 106 and/or other liquid sensors 134, the FLDL 200 may determine whether one or more single-threshold conditions are met (204). A single-threshold condition may include a condition or set of conditions that are dependent on one or more readings from a single liquid sensor. Accordingly, a single-threshold condition for a given fill-level within a flask may be met independently of any conditions that may be present at other fill-levels. To determine whether one or more single-threshold conditions are met, the FLDL 200 may compare the measured readings from the second liquid sensor 106 and/or other liquid sensors 134 to respective fill-level thresholds for the respective fill levels of the second liquid sensor 106 and/or other liquid sensors 134. A fill-level threshold may include a predetermined value selected to indicate liquid saturation at a given fill-level. Thus, when a measured reading from a liquid sensor for a given fill-level exceeds the corresponding predetermined value for that fill-level, the corresponding single-threshold condition is met.

After obtaining the measured readings from the second liquid sensor 106 and the first liquid sensor 104, the FLDL 200 may determine whether one or more multiple-threshold conditions are met (206). A multiple-threshold condition may include a set of conditions that are dependent at least one reading from each of multiple liquid sensors each placed to obtain measurements at different fill levels. Accordingly, a measured reading from a liquid sensor for a given fill-level exceeding the corresponding fill-level threshold may be insufficient to meet a multiple-threshold condition. Rather, the multiple-threshold condition may be met when a measured reading from a liquid sensor exceeds a corresponding fill-level threshold while measured readings from one or more other liquid sensors exceed corresponding supplemental thresholds. In various implementations, a supplemental threshold for a given fill level may be lower than the corresponding fill-level threshold. Thus, such example supplemental thresholds may indicate partial liquid saturation at fill levels where the corresponding liquid sensor they are exceeded.

In various implementations, a supplemental threshold for a given fill level may be about half the corresponding fill-level threshold for that fill level. For example, the supplemental threshold may be between 40% to 60% of the corresponding fill-level threshold. For example, the supplemental threshold may be between 43% to 50% of the corresponding fill-level threshold. For example, the supplemental threshold may be 44% of the corresponding fill-level threshold. For example, the supplemental threshold may be 49% of the corresponding fill-level threshold.

In an illustrative example, a multiple-threshold condition may include a condition that measured readings from the first sensor 104 exceed a fill-level threshold and measured readings from the second sensor 106 exceed a supplemental threshold.

Accordingly, the example multiple-threshold condition may be met when liquid saturation is present at the first fill level at the same time that partial saturation is present at the second fill level. As discussed above, single-threshold conditions may be met by liquid saturation being present at the corresponding fill-level. Accordingly, multiple-threshold conditions may be more stringent than some single threshold conditions.

A multiple-threshold condition may be implemented at fill levels where spurious measured readings (e.g., unduly high (or low) readings due to turbulence or other transient effects) may lead to a failure in the operation of the system that is using the flask. For example, fill levels for which multiple-threshold conditions may be implemented may include those where such failure would occur if the actual filling of the flask is lower (or higher) than that fill level.

When the one or more of the single-threshold conditions are met, the FLDL 200 may generate one or more fill-level indications indicating that the flask is filled to the corresponding fill-level (208). When the multiple-threshold condition is met, the FLDL 200 may generate a fill-level indications indicating that the flask is filled to the first fill-level (210).

In various implementations, the FLDL 200 may send the fill-level indication corresponding to the highest fill level for which conditions (either single-threshold or multiple-threshold conditions) are met. In some cases, fill-level indication corresponding to the highest fill level may be sent regardless of whether conditions are met at lower fill levels. In some cases, fill-level indications for the lower fill levels may be omitted (e.g., not generated because the FLDL 200 may be configured to skip testing at lower fill levels when conditions for higher fill levels are met). In some cases, fill-level indications for the lower fill levels may be deleted. In some cases, the fill-level indications for the lower fill levels may be suppressed (e.g., generated and stored but not send for display and/or recordation). In some cases, the fill-level indications for the lower fill levels may be overwritten (e.g., stored at the same memory location that the FLDL 200 will later write higher fill-level indications if corresponding conditions are met).

In various implementations, if no fill-level indication is generated (e.g., because none of the conditions are met), the FLDL 200 may generate an empty flask error. For example, the FLDL 200 may generate an empty flask error when the sensors are polled and no fill level indication is generated.

The fill-level indications and empty flask errors may be sent to memory for recording and/or a display for presentation to a user. The fill levels may be presented as fill percentages (or other portion identifiers) for ease or interpretation by users. For example, a flask with four fill levels (as shown in the example device 100) may have fill levels corresponding to 25%, 50%, 75%, and 100%. Similarly, a flask with three fill levels may have fill levels corresponding to 1/3 full, 2/3 full, and full.

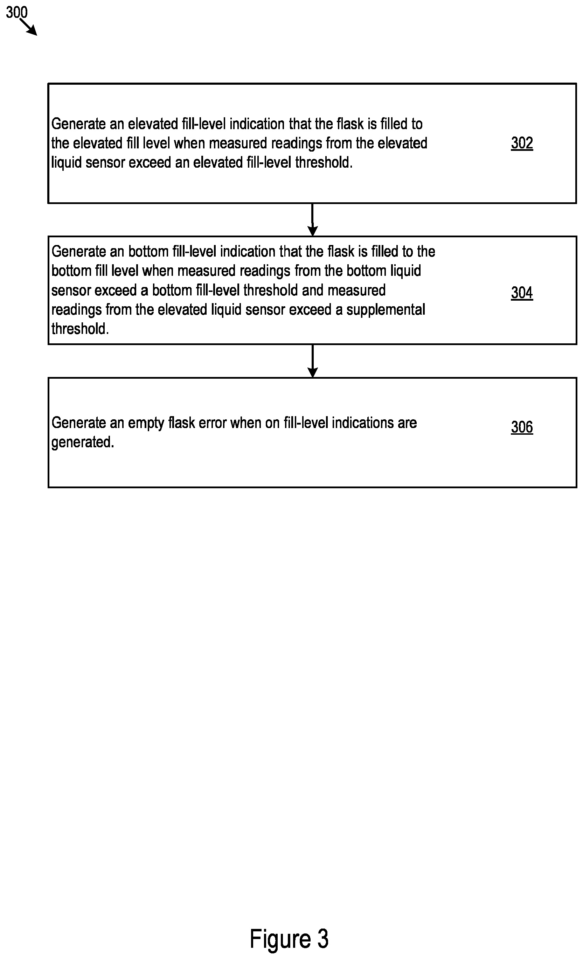

Referring now to Figure 3 while continuing to refer to Figure 1, second example FLDL 300 is shown. The second example FLDL 300 may also be executed (e.g., by the control circuitry 108). In various implementations, the second example FLDL 300 may also include various features of the first example FLDL 200. For example, the FLDL 300 may, in various implementations, poll sensors at intervals. For example, the FLDL 300 may, in various implementations, operate on a flask with two or more fill levels (e.g., at least a bottom fill level and one elevated fill level). The FLDL 300 may, in various implementations, operate with turbulence in the liquid within the flask. The FLDL 300 may, in various implementations, operate using measured readings that may be adjusted based on calibrated values and/or temperature-based adjustments.

In various implementations, the FLDL 300 generate an elevated fill-level indication that the flask is at the elevated fill level (e.g., the second fill level 116 or any one of the other fill levels 144) when measured readings from the elevated liquid sensor (e.g., the second liquid sensor 106 or any one of the other liquid sensors 134) exceed an elevated fill-level threshold (302).

The FLDL 300 may generate a bottom fill-level indication when measured readings from the bottom liquid sensor (e.g., the first liquid sensor 104) exceed a bottom fill-level threshold while measured readings from the elevated sensor exceed a supplementary threshold (304).

The FLDL 300 may generate an empty flask error when no fill-level indication is generated (306).

In various implementations, the FLDL 300 may suppress, delete, overwrite, or omit fill-level indications for lower levels when fill-level indications for higher levels are generated.

### Example Implementations

Various implementations have been specifically described. However, many other implementations are also possible. For example, the example implementations included below are described to be illustrative of various ones of the principles discussed above. However, the examples included below are not intended to be limiting, but rather, in some cases, specific examples to aid in the illustration of the above described techniques and architectures. The features of the following example implementations may be combined in various groupings in accord with the techniques and architectures describe above.

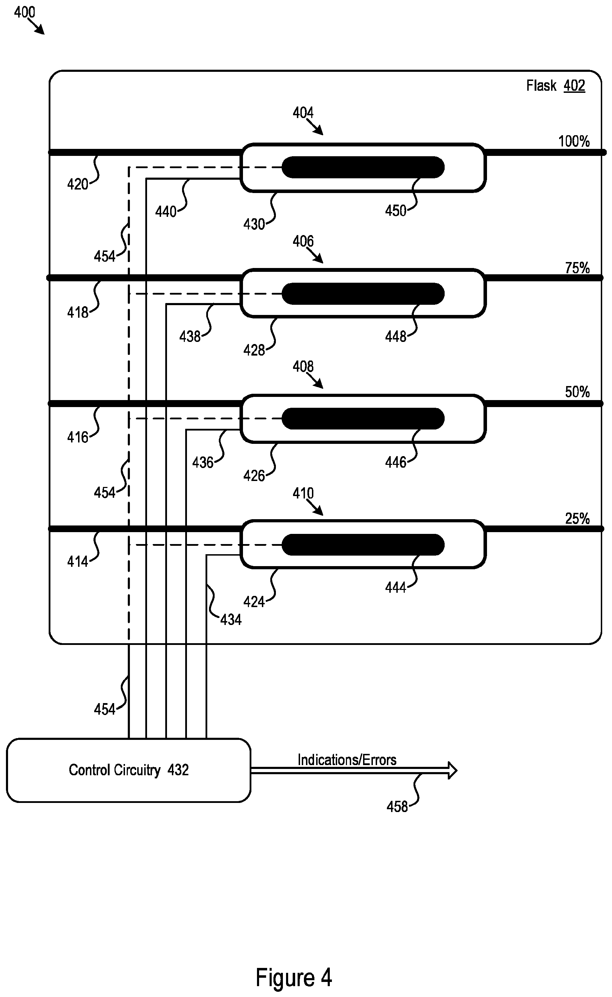

Referring to Figure 4, an example capacitive fill-level detection device 400 is shown. The example device 400 may include a flask 402. The device 400 may include four capacitive liquid sensors 404, 406, 408, 410 that correspond to fill levels 414, 416, 418, 420 within the flask. The four capacitive sensors may each include an outer electrode 424, 426, 428, and 430, which may be individually communicatively coupled to the control circuitry 432 via lines 434, 436, 438, and 440. The four capacitive sensors may each include an inner electrode 444, 446, 448, and 450, which may be on the same plane as the outer electrode (on the outer wall of the flask) and communicatively coupled to the control circuitry 432 as a group via line 454. Using logic (e.g., example logic 300), the control circuitry 432 may generate outputs 458 (e.g., based on measured readings from the capacitive liquid sensors 404, 406, 408, 410). The fill levels 414, 416, 418, 420 correspond to fill levels of 25%, 50%, 75%, and 100% respectively.

The inner electrodes may generate pulses (or other transient signals). The amount of energy that reaches the respective outer electrode from the pulse may depend on the liquid content at the corresponding fill level within the flask. The electrical coupling between the inner and outer electrodes is increased when the liquid content increases at the corresponding fill level within the flask which changes the capacitance of the sensor and increases the amount of energy transferred between the electrodes.

In various implementations, the capacitive liquid sensors 404, 406, 408, 410 may be based on commercially available touchscreen capacitive sensors.

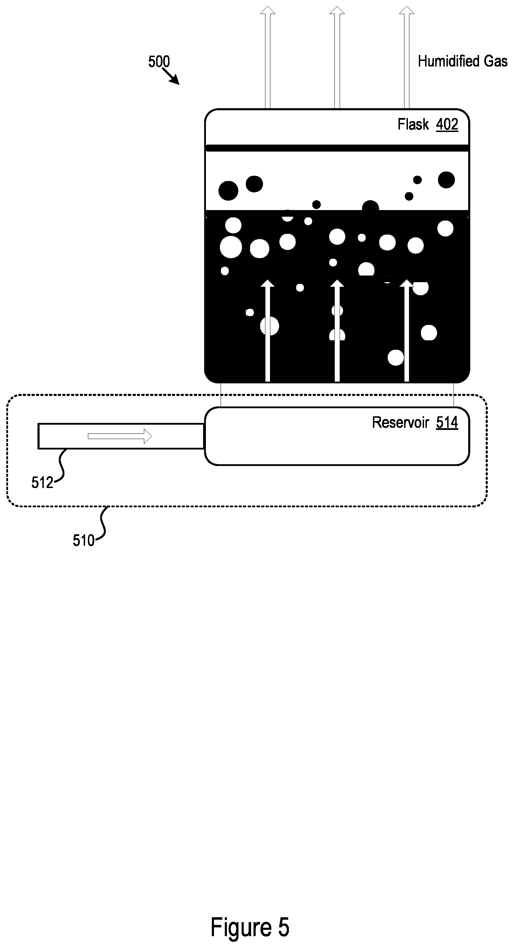

Figure 5 shows an example gas humidification system (GHS) 500 that may implement the example capacitive fill-level detection device 400. The example GHS 500 may include the fill-level detection device 400 (including the flask 402). The example GHS 500 may further include a gas injection channel 510. The gas injection channel may be coupled to the flask 402 to force a bubbling liquid-gas mixture upward into the flask 402.

The gas injection channel 510 may include a gas injection line 512 and a reservoir 514 coupled to the gas injection line 512. The reservoir 514 may be coupled to the flask 402 from the bottom. The reservoir 514 may hold a liquid (such as water) that may be force upward into the flask 402 when gas is sent into the reservoir 514 via the gas injection line 512.

The GHS 500 may humidify the injected gas via exposure of the gas to the liquid as it bubbles upward through the flask 402.

The GHS 500 may be used in various gas humidification systems. For example, the GHS 500 may be used to humidify gas used in an incubation system, such as embryonic incubators used in assistive reproductive technology (ART) systems.

In various applications, multiple gas humidification systems (such as the example GHS 500) may be implemented to operate in parallel within a unified device. The multiple gas humidification systems may operate identically or may differ from one another in flask structure and/or operative parameters (such as fill-level thresholds, temperature adjustments, supplemental thresholds, and/or other operative parameters).

The methods, devices, processing, and logic described above may be implemented in many different ways and in many different combinations of hardware and software. For example, all or parts of the implementations may be circuitry that includes an instruction processor, such as a Central Processing Unit (CPU), microcontroller, or a microprocessor; an Application Specific Integrated Circuit (ASIC), Programmable Logic Device (PLD), or Field Programmable Gate Array (FPGA); or circuitry that includes discrete logic or other circuit components, including analog circuit components, digital circuit components or both; or any combination thereof. The circuitry may include discrete interconnected hardware components and/or may be combined on a single integrated circuit die, distributed among multiple integrated circuit dies, or implemented in a Multiple Chip Module (MCM) of multiple integrated circuit dies in a common package, as examples.

The circuitry may further include or access instructions for execution by the circuitry. The instructions may be embodied as a signal and/or data stream and/or may be stored in a tangible storage medium that is other than a transitory signal, such as a flash memory, a Random Access Memory (RAM), a Read Only Memory (ROM), an Erasable Programmable Read Only Memory (EPROM); or on a magnetic or optical disc, such as a Compact Disc Read Only Memory (CDROM), Hard Disk Drive (HDD), or other magnetic or optical disk; or in or on another machine-readable medium. A product, such as a computer program product, may particularly include a storage medium and instructions stored in or on the medium, and the instructions when executed by the circuitry in a device may cause the device to implement any of the processing described above or illustrated in the drawings.

The implementations may be distributed as circuitry, e.g., hardware, and/or a combination of hardware and software among multiple system components, such as among multiple processors and memories, optionally including multiple distributed processing systems. Parameters, databases, and other data structures may be separately stored and managed, may be incorporated into a single memory or database, may be logically and physically organized in many different ways, and may be implemented in many different ways, including as data structures such as linked lists, hash tables, arrays, records, objects, or implicit storage mechanisms. Programs may be parts (e.g., subroutines) of a single program, separate programs, distributed across several memories and processors, or implemented in many different ways, such as in a library, such as a shared library (e.g., a Dynamic Link Library (DLL)). The DLL, for example, may store instructions that perform any of the processing described above or illustrated in the drawings, when executed by the circuitry.

## Claims

1. A device including:
a flask (102) for a liquid;
a first liquid sensor (104) placed to measure a first amount of liquid present at a first fill level within the flask;
a second liquid sensor (106) placed to measure a second amount of liquid present at a second fill level within the flask, the second liquid sensor farther from a bottom of the flask than the first liquid sensor; and
control circuitry (108) in operative communication with the first and second liquid sensors, the control circuitry configured to:
obtain a first measured reading from the first sensor, the first measured reading indicative of the first amount; and
obtain a second measured reading from the second sensor, the second measured reading indicative of the second amount; and
**characterized in that**, the control circuitry is further configured to
determine that one or more single-threshold conditions are met at a time that the second measured reading exceeds a second fill-level threshold;
determine that a multiple-threshold condition is met at a time that:
the first measured reading exceeds a first fill-level threshold; and
the second measured reading exceeds a supplemental threshold, the supplemental threshold different than the second fill-level threshold;
generate an elevated fill-level indication that the flask is filled at least to the second fill level at a time that the one or more single-threshold conditions are met; and
generate a first fill-level indication that the flask if filled to the first fill level at a time that the multiple-threshold condition is met.

2. The device of claim1, where:
the flask includes:
a third fill level; and
a fourth fill level; and
the control circuitry is configured to generate an elevated fill-level indication by
generating an indication that the flask is filled to the second fill level, the third fill level, or the fourth fill level, where:
optionally, the first, second, third, and fourth fill levels correspond to 25%, 50%, 75%, and 100% fill-level indications, respectively.

3. The device of claim 1 or 2, where the device further includes:
a third liquid senor placed to measure a third amount of liquid present at a third fill level within the flask, and where:
the control circuitry is further configured to:
determine that the one or more single-threshold conditions are met at a time that the third measured reading exceeds a third fill-level threshold; and
generate the elevated fill-level indication that the flask is filled at least to the third fill level at a time that:
the one or more single-threshold conditions are met; and
the third measured reading exceeds a third fill-level threshold, for example where the device further includes:
a fourth liquid senor placed to measure a fourth amount of liquid present at a fourth fill level within the flask, and where:
the control circuitry is further configured to:
determine that the one or more single-threshold conditions are met at a time that the fourth measured reading exceeds a fourth fill-level threshold; and
generate the elevated fill-level indication that the flask is filled at least to the fourth fill level at a time that:
the one or more single-threshold conditions are met; and
the fourth measured reading exceeds a fourth fill-level threshold.

4. The device of claim 1 or any other claim, where the control circuitry is configured to perform at least one of the following:
(a) generate an empty flask error at a time that no fill-level indication is generated;
(b) generate a highest fill-level indication for a highest fill level for which a single-threshold condition, a multiple-threshold condition or both are met regardless of whether conditions are met for one or more fill levels below the highest fill level;
(c) suppress a fill-level indication corresponding to any of the fill levels below the highest fill level;
(d) obtain the first measured reading by subtracting a calibrated value from a raw reading from the first liquid sensor;
(e) obtain the first measured reading by applying a temperature-dependent adjustment to a raw reading from the first liquid sensor;
(f) poll the liquid sensors at an interval, where:
optionally, the control circuitry is configured to generate an empty flask error at a time that the poll of the liquid sensors does not result in generation of a fill-level indication;
optionally, the interval includes:
a predetermined interval;
a periodic interval;
or both;
optionally, the interval is less than one minute;
optionally, the interval is less than twenty seconds; and
optionally, the interval is ten seconds.

5. The device of claim 1 or any other claim, where the flask is configured to hold the liquid in a turbulent state, for example where the turbulent state includes bubbling due to gas injection for humidification.

6. The device of claim 1 or any other claim, where supplemental threshold is set to about half of the second fill-level threshold, where:
optionally, the supplemental threshold is set between 40% to 60% of the second fill-level threshold;
optionally, the supplemental threshold is set between 43% to 50% of the second fill-level threshold;
optionally, the supplemental threshold is set 44% of the second fill-level threshold; and
optionally, the supplemental threshold is set 49% of the second fill-level threshold.

7. The device of claim 1, where the liquid sensors include a capacitive sensor, an optical sensor, an ionization sensor, or any grouping thereof and/or where the first and second liquid sensors are placed to support:
generation of error messages by the control circuitry where the flask filled below a minimum operational tolerance; or
generation of error messages by the control circuitry where the flask filled above a maximum operational tolerance.

8. A device including:
a flask;
a bottom liquid sensor corresponding to a bottom fill level of the flask;
an elevated liquid sensor corresponding to an elevated fill level of the flask, the elevated fill level above the bottom fill level; and
control circuitry in operative communication with the first and second liquid sensors, **characterized in that**, the control circuitry is configured to:
generate an elevated fill-level indication that the flask is at the elevated fill level at a time that a measured reading from the elevated liquid sensor exceeds an elevated fill-level threshold;
generate a bottom fill-level indication that the flask is at the bottom fill level at a time that:
a measured reading from the bottom liquid sensor exceeds a bottom fill-level threshold; and
the measured reading from the elevated liquid sensor exceeds a supplementary threshold, the supplementary threshold different than the elevated fill-level threshold; and
generate an empty flask error at a time that no fill-level indication is generated.

9. The device of claim 8 or any other claim, where the bottom fill level includes a lowest fill level for which the control circuitry is configured to generate a fill-level indication, and/or where the elevated fill level includes a fill level adjacent to the bottom fill level, and/or where the device includes one or more fill levels above the elevated fill level, and/or where the control circuitry is configured to suppress the bottom fill-level indication at a time that:
the measured reading from the bottom liquid sensor exceeds the bottom fill-level threshold; and
the measured reading from the elevated liquid sensor exceeds the elevated fill-level threshold.

10. A device including:
a flask;
a gas injection channel coupled to the flask to force a bubbling liquid-gas mixture upward into the flask;
a bottom capacitive sensor placed to measure a liquid content of the bubbling liquid-gas mixture at a bottom fill level of the flask;
a second fill-level capacitive sensor placed to measure a liquid content of the bubbling liquid-gas mixture at an second fill level of the flask, the second fill level above the bottom fill level and adjacent to the bottom fill level; and
control circuitry in operative communication with the bottom and second fill-level liquid sensors, the control circuitry configured to:
generate a second fill-level indication that the flask is at the second fill level at a time that the liquid content of the bubbling liquid-gas mixture at the second fill level exceeds a second fill-level threshold;
generate a bottom fill-level indication that the flask is at the bottom fill level at a time that:
the liquid content of the bubbling liquid-gas mixture at the bottom fill level exceeds a bottom fill-level threshold; and
the liquid content of the bubbling liquid-gas mixture at the second fill level exceeds a supplementary threshold, the supplementary threshold different than the second fill-level threshold; and
generate an empty flask error at a time that no fill-level indication is generated.

11. The device of claim 10 or any other claim, where the gas injection channel includes:
a gas injection line; and
a reservoir coupled to the gas injection line, the reservoir coupled to and positioned below the flask to force the bubbling liquid-gas mixture upward into the flask, and/or where the flask includes a humidification flask to humidify a gas of the bubbling liquid-gas mixture through exposure to the liquid content, for example where the liquid content includes water content, for example where the device is included within a gas humidification system of an incubator, where:
optionally, the gas humidification system includes multiple parallel humidification flasks including the humidification flask;
optionally, the multiple parallel humidification flasks differ in configuration and one or more operational parameters;
optionally, the incubator is included within an assistive reproductive technology (ART) system.

12. A method including:
obtaining a first measured reading from a first liquid sensor, the first measured reading indicative of a first amount of liquid present at a first fill-level of a flask;
obtaining a second measured reading from a second liquid sensor, the second measured reading indicative of a second amount of liquid present at a second fill-level of a flask, the second liquid sensor corresponds to the second-level fill sensor for which the first liquid sensor is positioned below;
with a control circuitry (108) in operative communication with the first and second liquid sensors,
**characterized in that**,
the control circuitry is configured in determining that one or more single-threshold conditions are met at a time that the second measured reading exceeds a second fill-level threshold;
determining that a multiple-threshold condition is met at a time that:
the first measured reading exceeds a first fill-level threshold; and
the second measured reading exceeds a supplemental threshold, the supplemental threshold different than the second fill-level threshold;
generating an elevated fill-level indication that the flask is filled to at least the second fill level at a time that the one or more single-threshold conditions are met; and
generating a first fill-level indication that the flask if filled to the first fill level at a time that the multiple-threshold condition is met.

13. The method of claim 12 for a device of claim 10 or 11, further including one of:
(a) at a time when the one or more single-threshold conditions are met and the multiple-threshold condition is met, omitting the first fill-level indication
(b) causing turbulence in the liquid present in the flask by forcing gas upward through the bottom of the flask
(c) polling the liquid sensors at an interval; and
generating a fill-level indication or an empty flask error at each interval.

14. The method of claim 13 or any of the other claims, where:
obtaining the first measured reading and the second measured reading includes one of:
(a) subtracting a first calibrated value from a first raw reading from the first liquid sensor for obtaining the first measured reading; and
subtracting a second calibrated value from a second raw reading from the second liquid sensor for obtaining the first measured reading; and
(b) applying a first temperature-dependent adjustment to a first raw reading from the first liquid sensor for obtaining the first measured reading; and
applying a second temperature-dependent adjustment to a second raw reading from the second liquid sensor for obtaining the second measured reading.

15. A method including:
generating an elevated fill-level indication that a flask is at an elevated fill level at a time that a measured reading from an elevated liquid sensor exceeds an elevated fill-level threshold, the elevated liquid sensor positioned to correspond to an elevated fill-level of flask, for which a bottom liquid sensor is positioned below;
with a control circuitry (108) in operative communication with the bottom liquid sensor and the elevated liquid sensor,
**characterized in that**,
the control circuitry is configured in generating a bottom fill-level indication that the flask is at the bottom fill level at a time that:
a measured reading from the bottom liquid sensor exceeds a bottom fill-level threshold; the bottom liquid sensor positioned to correspond to an bottom fill-level of a flask; and
the measured reading from the elevated liquid sensor exceeds a supplementary threshold, the supplementary threshold different than the elevated fill-level threshold; and
generating an empty flask error at a time that no fill-level indication is generated.

## Patentansprüche

1. Vorrichtung, einschließend:
eine Flasche (102) für eine Flüssigkeit;
einen ersten Flüssigkeitssensor (104), der platziert ist, um eine erste Menge einer Flüssigkeit zu messen, die auf einem ersten Füllstand innerhalb der Flasche vorliegt;
einen zweiten Flüssigkeitssensor (106), der platziert ist, um eine zweite Menge einer Flüssigkeit zu messen, die auf einem zweiten Füllstand innerhalb der Flasche vorliegt, wobei der zweite Flüssigkeitssensor von einem Boden der Flasche weiter entfernt ist als der erste Flüssigkeitssensor; und
eine Steuerschaltung (108) in operativer Kommunikation mit dem ersten und dem zweiten Flüssigkeitssensor, wobei die Steuerschaltung konfiguriert ist zum:
Beziehen eines ersten gemessenen Ablesewerts von dem ersten Sensor, wobei der erste gemessene Ablesewert die erste Menge angibt; und
Beziehen eines zweiten gemessenen Ablesewerts von dem zweiten Sensor, wobei der zweite gemessene Ablesewert die zweite Menge angibt; und **dadurch gekennzeichnet, dass** die Steuerschaltung ferner konfiguriert ist, um zu bestimmen, dass eine oder mehrere Bedingungen mit einem einzigen Schwellenwert zu einer Zeit erfüllt sind, zu der der zweite gemessene Ablesewert einen zweiten Füllstand-Schwellenwert übersteigt;
Bestimmen, dass eine Bedingung mit mehreren Schwellenwerten zu einer Zeit erfüllt ist, zu der:
der erste gemessene Ablesewert einen ersten Füllstand-Schwellenwert übersteigt; und
der zweite gemessene Ablesewert einen zusätzlichen Schwellenwert übersteigt, wobei sich der zusätzliche Schwellenwert von dem zweiten Füllstand-Schwellenwert unterscheidet;
Erzeugen einer Erhöhter-Füllstand-Angabe, dass die Flasche mindestens zu dem zweiten Füllstand zu einer Zeit gefüllt ist, zu der die eine oder die mehreren Bedingungen mit einem einzigen Schwellenwert erfüllt sind; und
Erzeugen einer Erster-Füllstand-Angabe, dass die Flasche mindestens zu dem ersten Füllstand zu einer Zeit gefüllt ist, zu der die Bedingung mit mehreren Schwellenwerten erfüllt ist.

2. Vorrichtung nach Anspruch 1, wobei:
die Flasche einschließt:
einen dritten Füllstand; und
einen vierten Füllstand; und
die Steuerschaltung konfiguriert ist, um eine Erhöhter-Füllstand-Angabe durch Erzeugen einer Angabe zu erzeugen, dass die Flasche zu dem zweiten Füllstand, dem dritten Füllstand oder dem vierten Füllstand gefüllt ist, wobei:
optional der erste, der zweite, der dritte und der vierte Füllstand einer 25-%-, 50-%-, 75-%- bzw. 100-%-Füllstand-Angabe entsprechen.

3. Vorrichtung nach Anspruch 1 oder 2, wobei die Vorrichtung ferner einschließt:
einen dritten Flüssigkeitssensor, der platziert ist, um eine dritte Menge einer Flüssigkeit zu messen, die auf einem dritten Füllstand innerhalb der Flasche vorliegt, und wobei:
die Steuerschaltung ferner konfiguriert ist zum:
Bestimmen, dass die eine oder die mehreren Bedingungen mit einem einzigen Schwellenwert zu einer Zeit erfüllt sind, zu der der dritte gemessene Ablesewert einen dritten Füllstand-Schwellenwert übersteigt; und
Erzeugen der Erhöhter-Füllstand-Angabe, dass die Flasche mindestens zu dem dritten Füllstand zu einer Zeit gefüllt ist, zu der:
die eine oder die mehreren Bedingungen mit einem einzigen Schwellenwert erfüllt sind; und
der dritte gemessene Ablesewert einen dritten Füllstand-Schwellenwert übersteigt, beispielsweise wobei die Vorrichtung ferner einschließt:
einen vierten Flüssigkeitssensor, der platziert ist, um eine vierte Menge einer Flüssigkeit zu messen, die auf einem vierten Füllstand innerhalb der Flasche vorliegt, und wobei:
die Steuerschaltung ferner konfiguriert ist zum:
Bestimmen, dass die eine oder die mehreren Bedingungen mit einem einzigen Schwellenwert zu einer Zeit erfüllt sind, zu der der vierte gemessene Ablesewert einen vierten Füllstand-Schwellenwert übersteigt; und
Erzeugen der Erhöhter-Füllstand-Angabe, dass die Flasche mindestens zu dem vierten Füllstand zu einer Zeit gefüllt ist, zu der:
die eine oder die mehreren Bedingungen mit einem einzigen Schwellenwert erfüllt sind; und
der vierte gemessene Ablesewert einen vierten Füllstand-Schwellenwert übersteigt.

4. Vorrichtung nach Anspruch 1 oder einem beliebigen anderen Anspruch, wobei die Steuerschaltung konfiguriert ist, um mindestens eines der folgenden durchzuführen:
(a) Erzeugen eines Leere-Flasche-Fehlers zu einer Zeit, zu der keine Füllstand-Angabe erzeugt wird;
(b) Erzeugen einer Höchster-Füllstand-Angabe für einen höchsten Füllstand, für den eine Bedingung mit einem einzigen Schwellenwert, eine Bedingung mit mehreren Schwellenwerten oder beide erfüllt sind, ungeachtet dessen, ob Bedingungen für einen oder mehrere Füllstände unterhalb des höchsten Füllstands erfüllt sind;
(c) Unterdrücken einer Füllstand-Angabe, die einem beliebigen der Füllstände unterhalb des höchsten Füllstands entspricht;
(d) Beziehen des ersten gemessenen Ablesewerts durch Subtrahieren eines kalibrierten Werts von einem rohen Ablesewert von dem ersten Flüssigkeitssensor;
(e) Beziehen des ersten gemessenen Ablesewerts durch Anwenden einer temperaturabhängigen Justierung auf den rohen Ablesewert von dem ersten Flüssigkeitssensor;
(f) Abfragen der Flüssigkeitssensoren in einem Intervall, wobei:
optional die Steuerschaltung konfiguriert ist, um einen Leere-Flasche-Fehler zu einer Zeit zu erzeugen, zu der die Abfrage der Flüssigkeitssensoren nicht zu einer Erzeugung einer Füllstand-Angabe führt;
optional das Intervall einschließt:
ein vorbestimmtes Intervall;
ein periodisches Intervall;
oder beides;
optional das Intervall weniger als eine Minute beträgt;
optional das Intervall weniger als zwanzig Sekunden beträgt; und
optional das Intervall zehn Sekunden beträgt.

5. Vorrichtung nach Anspruch 1 oder einem beliebigen anderen Anspruch, wobei die Flasche konfiguriert ist, um die Flüssigkeit in einem turbulenten Zustand zu halten, beispielsweise wobei der turbulente Zustand ein Sprudeln aufgrund einer Gasinjektion zur Befeuchtung einschließt.

6. Vorrichtung nach Anspruch 1 oder einem beliebigen anderen Anspruch, wobei ein zusätzlicher Schwellenwert auf etwa die Hälfte des zweiten Füllstand-Schwellenwerts eingestellt wird, wobei:
optional der zusätzliche Schwellenwert zwischen 40 % bis 60 % des zweiten Füllstand-Schwellenwerts eingestellt wird;
optional der zusätzliche Schwellenwert zwischen 43 % bis 50 % des zweiten Füllstand-Schwellenwerts eingestellt wird;
optional der zusätzliche Schwellenwert auf 44 % des zweiten Füllstand-Schwellenwerts eingestellt wird; und
optional der zusätzliche Schwellenwert auf 49 % des zweiten Füllstand-Schwellenwerts eingestellt wird.

7. Vorrichtung nach Anspruch 1, wobei die Flüssigkeitssensoren einen kapazitiven Sensor, einen optischen Sensor, einen Ionisationssensor oder eine beliebige Gruppierung davon einschließen und/oder wobei der erste und der zweite Flüssigkeitssensor platziert sind zum Unterstützen von:
Erzeugung von Fehlernachrichten durch die Steuerschaltung, wenn die Flasche unterhalb einer minimalen Betriebstoleranz gefüllt ist; oder
Erzeugung von Fehlernachrichten durch die Steuerschaltung, wenn die Flasche oberhalb einer maximalen Betriebstoleranz gefüllt ist.

8. Vorrichtung, einschließend:
eine Flasche;
einen unteren Flüssigkeitssensor, der einem unteren Füllstand der Flasche entspricht;
einen erhöhten Flüssigkeitssensor, der einem erhöhten Füllstand der Flasche entspricht, wobei der erhöhte Füllstand oberhalb des unteren Füllstands ist; und
eine Steuerschaltung in operativer Kommunikation mit dem ersten und dem zweiten Flüssigkeitssensor, **dadurch gekennzeichnet, dass** die Steuerschaltung konfiguriert ist zum: Erzeugen einer Erhöhter-Füllstand-Angabe, dass die Flasche auf dem erhöhten Füllstand zu einer Zeit ist, zu der ein gemessener Ablesewert von dem erhöhten Flüssigkeitssensor einen Erhöhter-Füllstand-Schwellenwert übersteigt;
Erzeugen der Unterer-Füllstand-Angabe, dass die Flasche auf dem unteren Füllstand zu einer Zeit ist, zu der:
ein gemessener Ablesewert von dem unteren Flüssigkeitssensor einen Unterer-Füllstand-Schwellenwert übersteigt; und
der gemessene Ablesewert von dem erhöhten Flüssigkeitssensor einen zusätzlichen Schwellenwert übersteigt, wobei sich der zusätzliche Schwellenwert von dem Erhöhter-Füllstand-Schwellenwert unterscheidet; und
Erzeugen eines Leere-Flasche-Fehlers zu einer Zeit, zu der keine Füllstand-Angabe erzeugt wird.

9. Vorrichtung nach Anspruch 8 oder einem beliebigen anderen Anspruch, wobei der untere Füllstand einen untersten Füllstand einschließt, für den die Steuerschaltung konfiguriert ist, um eine Füllstand-Angabe zu erzeugen, und/oder wobei der erhöhte Füllstand einen Füllstand angrenzend an den unteren Füllstand einschließt, und/oder wobei die Vorrichtung einen oder mehrere Füllstände oberhalb des erhöhten Füllstands einschließt, und/oder wobei die Steuerschaltung konfiguriert ist, um die Unterer-Füllstand-Angabe zu einer Zeit zu unterdrücken, zu der:
ein gemessener Ablesewert von dem unteren Flüssigkeitssensor einen Unterer-Füllstand-Schwellenwert übersteigt; und
der gemessene Ablesewert von dem erhöhten Flüssigkeitssensor den Erhöhter-Füllstand-Schwellenwert übersteigt.

10. Vorrichtung, einschließend:
eine Flasche;
einen Gasinjektionskanal, der an die Flasche gekoppelt ist, um ein sprudelndes Flüssigkeit-Gas-Gemisch nach oben in die Flasche zu treiben;
einen unteren kapazitiven Sensor, der platziert ist, um einen Flüssigkeitsinhalt des sprudelnden Flüssigkeit-Gas-Gemischs auf einem unteren Füllstand der Flasche zu messen;
einen zweiten kapazitiven Füllstandsensor, der platziert ist, um einen Flüssigkeitsinhalt des sprudelnden Flüssigkeit-Gas-Gemischs auf einem zweiten Füllstand der Flasche zu messen, wobei der zweite Füllstand oberhalb des unteren Füllstands und angrenzend an den unteren Füllstand ist; und
eine Steuerschaltung in operativer Kommunikation mit dem unteren und dem zweiten Füllstand-Flüssigkeitssensor, wobei die Steuerschaltung konfiguriert ist zum:
Erzeugen einer Zweiter-Füllstand-Angabe, dass die Flasche auf dem zweiten Füllstand zu einer Zeit ist, zu der der Flüssigkeitsinhalt des sprudelnden Flüssigkeit-Gas-Gemischs auf dem zweiten Füllstand einen Zweiter-Füllstand-Schwellenwert übersteigt;
Erzeugen der Unterer-Füllstand-Angabe, dass die Flasche auf dem unteren Füllstand zu einer Zeit ist, zu der:
der Flüssigkeitsinhalt des sprudelnden Flüssigkeit-Gas-Gemischs auf dem unteren Füllstand einen Unterer-Füllstand-Schwellenwert übersteigt; und
der Flüssigkeitsinhalt des sprudelnden Flüssigkeit-Gas-Gemischs auf dem zweiten Füllstand einen zusätzlichen Schwellenwert übersteigt, wobei sich der zusätzliche Schwellenwert von dem Zweiter-Füllstand-Schwellenwert unterscheidet; und
Erzeugen eines Leere-Flasche-Fehlers zu einer Zeit, zu der keine Füllstand-Angabe erzeugt wird.

11. Vorrichtung nach Anspruch 10 oder einem beliebigen anderen Anspruch, wobei der Gasinjektionskanal einschließt:
eine Gasinjektionsleitung; und
ein Reservoir, das an die Gasinjektionsleitung gekoppelt ist, wobei das Reservoir an die Flasche gekoppelt ist und unterhalb dieser positioniert ist, um das sprudelnde Flüssigkeit-Gas-Gemisch nach oben in die Flasche zu treiben, und/oder wobei die Flasche eine Befeuchtungsflasche einschließt, um ein Gas des sprudelnden Flüssigkeit-Gas-Gemischs durch Aussetzen gegenüber dem Flüssigkeitsinhalt zu befeuchten, beispielsweise wobei der Flüssigkeitsinhalt Wasserinhalt einschließt, beispielsweise wobei die Vorrichtung innerhalb eines Gasbefeuchtungssystems eines Inkubators eingeschlossen ist, wobei:
optional das Gasbefeuchtungssystem mehrere parallele Befeuchtungsflaschen einschließt, einschließend die Befeuchtungsflasche;
optional sich die mehreren parallelen Befeuchtungsflaschen in Bezug auf Konfiguration und einen oder mehrere Betriebsparameter unterscheiden;
optional der Inkubator innerhalb eines Systems für assistierte Reproduktionstechnologie (ART) eingeschlossen ist.

12. Verfahren, einschließend:
Beziehen eines ersten gemessenen Ablesewerts von einem ersten Flüssigkeitssensor, wobei der erste gemessene Ablesewert eine erste Menge einer Flüssigkeit angibt, die auf einem ersten Füllstand einer Flasche vorliegt;
Beziehen eines zweiten gemessenen Ablesewerts von einem zweiten Flüssigkeitssensor, wobei der zweite gemessene Ablesewert eine zweite Menge einer Flüssigkeit angibt, die auf einem zweiten Füllstand einer Flasche vorliegt, wobei der zweite Flüssigkeitssensor dem Füllsensor des zweiten Pegels entspricht, für den der ersten Flüssigkeitssensor unterhalb positioniert ist; mit einer Steuerschaltung (108) in operativer Kommunikation mit dem ersten und dem zweiten Flüssigkeitssensor, **dadurch gekennzeichnet, dass** die Steuerschaltung zum Bestimmen konfiguriert ist, dass eine oder mehrere Bedingungen mit einem einzigen Schwellenwert zu einer Zeit erfüllt sind, zu der der zweite gemessene Ablesewert einen Zweiter-Füllstand-Schwellenwert übersteigt;
Bestimmen, dass eine Bedingung mit mehreren Schwellenwerten zu einer Zeit erfüllt ist, zu der:
der erste gemessene Ablesewert einen ersten Füllstand-Schwellenwert übersteigt; und
der zweite gemessene Ablesewert einen zusätzlichen Schwellenwert übersteigt, wobei sich der zusätzliche Schwellenwert von dem zweiten Füllstand-Schwellenwert unterscheidet;
Erzeugen einer Erhöhter-Füllstand-Angabe, dass die Flasche zu mindestens dem zweiten Füllstand zu einer Zeit gefüllt ist, zu der die eine oder die mehreren Bedingungen mit einem einzigen Schwellenwert erfüllt sind; und
Erzeugen einer Erster-Füllstand-Angabe, dass die Flasche mindestens zu dem ersten Füllstand zu einer Zeit gefüllt ist, zu der die Bedingung mit mehreren Schwellenwerten erfüllt ist.

13. Verfahren nach Anspruch 12 für eine Vorrichtung nach Anspruch 10 oder 11, ferner einschließend eines von:
(a) zu einer Zeit, zu der die eine oder die mehreren Bedingungen mit einem einzigen Schwellenwert erfüllt sind und die Bedingung mit mehreren Schwellenwerten erfüllt ist, Auslassen der Erster-Füllstand-Angabe (b) Bewirken von Turbulenz in der Flüssigkeit, die in der Flasche vorliegt, durch Treiben von Gas nach oben durch den Boden der Flasche (c) Abfragen der Flüssigkeitssensoren in einem Intervall; und
Erzeugen einer Füllstand-Angabe oder eines Leere-Flasche-Fehlers zu jedem Intervall.

14. Verfahren nach Anspruch 13 oder einem beliebigen der anderen Ansprüche, wobei:
das Beziehen des ersten gemessenen Ablesewerts und des zweiten gemessenen Ablesewerts eines der folgenden einschließt:
(a) Subtrahieren eines ersten kalibrierten Werts von einem ersten rohen Ablesewert von dem ersten Flüssigkeitssensor zum Beziehen des ersten gemessenen Ablesewerts; und
Subtrahieren eines zweiten kalibrierten Werts von einem zweiten rohen Ablesewert von dem zweiten Flüssigkeitssensor zum Beziehen des ersten gemessenen Ablesewerts; und
(b) Anwenden einer ersten temperaturabhängigen Justierung auf einen ersten rohen Ablesewert von dem ersten Flüssigkeitssensor zum Beziehen des ersten gemessenen Ablesewerts; und
Anwenden einer zweiten temperaturabhängigen Justierung auf einen zweiten rohen Ablesewert von dem zweiten Flüssigkeitssensor zum Beziehen des zweiten gemessenen Ablesewerts.

15. Verfahren, einschließend:
Erzeugen einer Erhöhter-Füllstand-Angabe, dass eine Flasche auf einem erhöhten Füllstand zu einer Zeit ist, zu der ein gemessener Ablesewert von einem erhöhten Flüssigkeitssensor einen Erhöhter-Füllstand-Schwellenwert übersteigt, wobei der erhöhte Flüssigkeitssensor positioniert ist, um einem erhöhten Füllstand einer Flasche zu entsprechen, für die ein unterer Flüssigkeitssensor unterhalb positioniert ist; mit einer Steuerschaltung (108) in operativer Kommunikation mit dem unteren Flüssigkeitssensor und dem erhöhten Flüssigkeitssensor, **dadurch gekennzeichnet, dass** die Steuerschaltung zum Erzeugen einer Unterer-Füllstand-Angabe konfiguriert ist, dass die Flasche auf dem unteren Füllstand zu einer Zeit ist, zu der:
ein gemessener Ablesewert von dem unteren Flüssigkeitssensor einen Unterer-Füllstand-Schwellenwert übersteigt; wobei der untere Flüssigkeitssensor positioniert ist, um einem unteren Füllstand einer Flasche zu entsprechen; und
der gemessene Ablesewert von dem erhöhten Flüssigkeitssensor einen zusätzlichen Schwellenwert übersteigt, wobei sich der zusätzliche Schwellenwert von dem Erhöhter-Füllstand-Schwellenwert unterscheidet;
und Erzeugen eines Leere-Flasche-Fehlers zu einer Zeit, zu der keine Füllstand-Angabe erzeugt wird.

## Revendications

1. Dispositif comportant :
un ballon (102) pour un liquide ;
un premier capteur de liquide (104) placé pour mesurer une première quantité de liquide présente à un premier niveau de remplissage à l'intérieur du ballon ;
un deuxième capteur de liquide (106) placé pour mesurer une deuxième quantité de liquide présente à un deuxième niveau de remplissage à l'intérieur du ballon, le deuxième capteur de liquide étant plus éloigné du fond du ballon que le premier capteur de liquide ; et
une circuiterie de commande (108) en communication fonctionnelle avec les premier et deuxième capteurs de liquide, la circuiterie de commande étant configurée pour :
obtenir une première lecture mesurée à partir du premier capteur, la première lecture mesurée indiquant la première quantité ; et
obtenir une deuxième lecture mesurée à partir du deuxième capteur, la deuxième lecture mesurée indiquant la deuxième quantité ; et **caractérisé en ce que** la circuiterie de commande est en outre configurée pour déterminer qu'une ou plusieurs conditions de seuil unique sont remplies à un moment où la deuxième lecture mesurée dépasse un deuxième seuil de niveau de remplissage ;
déterminer qu'une condition de seuils multiples est remplie à un moment où :
la première lecture mesurée dépasse un premier seuil de niveau de remplissage ; et
la deuxième lecture mesurée dépasse un seuil supplémentaire, le seuil supplémentaire étant différent du deuxième seuil de niveau de remplissage ;
générer une indication de niveau de remplissage élevé indiquant que le ballon est rempli au moins jusqu'au deuxième niveau de remplissage à un moment où la ou les conditions de seuil unique sont remplies ; et
générer une indication de premier niveau de remplissage indiquant que le ballon est rempli jusqu'au premier niveau de remplissage à un moment où la condition de seuils multiples est remplie.

2. Dispositif selon la revendication 1, dans lequel :
le ballon comporte :
un troisième niveau de remplissage ; et
un quatrième niveau de remplissage ; et
la circuiterie de commande est configurée pour générer une indication de niveau de remplissage élevé en générant une indication indiquant que le ballon est rempli jusqu'au deuxième niveau de remplissage, jusqu'au troisième niveau de remplissage, ou jusqu'au quatrième niveau de remplissage, dans lequel :
facultativement, les premier, deuxième, troisième et quatrième niveaux de remplissage correspondent respectivement à des indications de niveau de remplissage de 25 %, 50 %, 75 % et 100 %.

3. Dispositif selon la revendication 1 ou 2, dans lequel le dispositif comporte en outre :
un troisième capteur de liquide placé pour mesurer une troisième quantité de liquide présente à un troisième niveau de remplissage à l'intérieur du ballon, et dans lequel :
la circuiterie de commande est en outre configurée pour :
déterminer que la ou les conditions de seuil unique sont remplies à un moment où la troisième lecture mesurée dépasse un troisième seuil de niveau de remplissage ; et
générer l'indication de niveau de remplissage élevé indiquant que le ballon est rempli au moins jusqu'au troisième niveau de remplissage à un moment où :
la ou les conditions de seuil unique sont remplies ; et
la troisième lecture mesurée dépasse un troisième seuil de niveau de remplissage, par exemple lorsque le dispositif comporte en outre :
un quatrième capteur de liquide placé pour mesurer une quatrième quantité de liquide présente à un quatrième niveau de remplissage à l'intérieur du ballon, et dans lequel :
la circuiterie de commande est en outre configurée pour :
déterminer que la ou les conditions de seuil unique sont remplies à un moment où la quatrième lecture mesurée dépasse un quatrième seuil de niveau de remplissage ; et
générer l'indication de niveau de remplissage élevé indiquant que le ballon est rempli au moins jusqu'au quatrième niveau de remplissage à un moment où :
la ou les conditions de seuil unique sont remplies ; et
la quatrième valeur mesurée dépasse un quatrième seuil de niveau de remplissage.

4. Dispositif selon la revendication 1 ou toute autre revendication, dans lequel la circuiterie de commande est configurée pour effectuer au moins l'un parmi :
(a) générer une erreur de ballon vide à un moment où aucune indication de niveau de remplissage n'est générée ;
(b) générer une indication de niveau de remplissage le plus élevé pour un niveau de remplissage le plus élevé pour lequel une condition de seuil unique, une condition de seuils multiples ou les deux sont remplies, indépendamment du fait que des conditions soient remplies ou non pour un ou plusieurs niveaux de remplissage en dessous du niveau de remplissage le plus élevé ;
(c) supprimer une indication de niveau de remplissage correspondant à l'un quelconque des niveaux de remplissage en dessous du niveau de remplissage le plus élevé ;
(d) obtenir la première lecture mesurée en soustrayant une valeur étalonnée d'une lecture brute provenant du premier capteur de liquide ;
(e) obtenir la première lecture mesurée en appliquant un ajustement dépendant de la température à une lecture brute provenant du premier capteur de liquide ;
(f) interroger les capteurs de liquide à un intervalle, dans lequel :
facultativement, la circuiterie de commande est configurée pour générer une erreur de ballon vide à un moment où l'interrogation des capteurs de liquide n'entraîne pas la génération d'une indication de niveau de remplissage ;
facultativement, l'intervalle comporte :
un intervalle prédéterminé ;
un intervalle périodique ;
ou les deux ;
facultativement, l'intervalle est inférieur à une minute ;
facultativement, l'intervalle est inférieur à vingt secondes ; et
facultativement, l'intervalle est de dix secondes.

5. Dispositif selon la revendication 1 ou toute autre revendication, dans lequel le ballon est configuré pour maintenir le liquide dans un état turbulent, par exemple dans lequel l'état turbulent inclut un bouillonnement dû à l'injection de gaz pour l'humidification.

6. Dispositif selon la revendication 1 ou toute autre revendication, dans lequel le seuil supplémentaire est fixé à environ la moitié du deuxième seuil de niveau de remplissage, dans lequel :
facultativement, le seuil supplémentaire est fixé entre 40 % et 60 % du deuxième seuil de remplissage ;
facultativement, le seuil supplémentaire est fixé entre 43 % et 50 % du deuxième seuil de remplissage ;
facultativement, le seuil supplémentaire est fixé à 44 % du deuxième seuil de remplissage ; et
facultativement, le seuil supplémentaire est fixé à 49 % du deuxième seuil de niveau de remplissage.

7. Dispositif selon la revendication 1, dans lequel les capteurs de liquide comportent un capteur capacitif, un capteur optique, un capteur à ionisation, ou tout groupe de ceux-ci et/ou dans lequel les premier et deuxième capteurs de liquide sont placés pour prendre en charge :
la génération de messages d'erreur par la circuiterie de commande lorsque le ballon s'est rempli en dessous d'une tolérance opérationnelle minimale ; ou
la génération de messages d'erreur par la circuiterie de commande lorsque le ballon s'est rempli au-dessus d'une tolérance opérationnelle maximale.

8. Dispositif comportant :
un ballon ;
un capteur de liquide de fond correspondant à un niveau de remplissage de fond du ballon ;
un capteur de liquide élevé correspondant à un niveau de remplissage élevé du ballon, le niveau de remplissage élevé étant au-dessus du niveau de remplissage de fond ; et
une circuiterie de commande en communication fonctionnelle avec les premier et deuxième capteurs de liquide, **caractérisé en ce que** la circuiterie de commande est configurée pour : générer une indication de niveau de remplissage élevé indiquant que le ballon est au niveau de remplissage élevé à un moment où une lecture mesurée à partir du capteur de liquide élevé dépasse un seuil de niveau de remplissage élevé ;
générer une indication du niveau de remplissage de fond indiquant que le ballon est au niveau de remplissage de fond à un moment où :
une lecture mesurée à partir du capteur de liquide de fond dépasse un seuil de niveau de remplissage de fond ; et
la lecture mesurée à partir du capteur de liquide élevé dépasse un seuil supplémentaire, le seuil supplémentaire étant différent du seuil de niveau de remplissage élevé ; et
générer une erreur de ballon vide à un moment où aucune indication de niveau de remplissage n'est générée.

9. Dispositif selon la revendication 8 ou toute autre revendication, dans lequel le niveau de remplissage de fond comporte un niveau de remplissage le plus bas pour lequel la circuiterie de commande est configurée pour générer une indication de niveau de remplissage, et/ou dans lequel le niveau de remplissage élevé comporte un niveau de remplissage adjacent au niveau de remplissage de fond, et/ou dans lequel le dispositif comporte un ou plusieurs niveaux de remplissage au-dessus du niveau de remplissage élevé, et/ou dans lequel la circuiterie de commande est configurée pour supprimer l'indication de niveau de remplissage de fond à un moment où :
la lecture mesurée à partir du capteur de liquide de fond dépasse le seuil de niveau de remplissage de fond ; et
la lecture mesurée à partir du capteur de liquide élevé dépasse le seuil de niveau de remplissage élevé.

10. Dispositif comportant :
un ballon ;
un canal d'injection de gaz accouplé au ballon pour forcer un mélange liquide-gaz bouillonnant vers le haut dans le ballon ;
un capteur capacitif de fond placé pour mesurer un contenu de liquide du mélange liquide-gaz bouillonnant à un niveau de remplissage de fond du ballon ;
un second capteur capacitif de niveau de remplissage placé pour mesurer un contenu de liquide du mélange liquide-gaz bouillonnant à un second niveau de remplissage du ballon, le second niveau de remplissage étant situé au-dessus du niveau de remplissage de fond et adjacent au niveau de remplissage de fond ; et
une circuiterie de commande en communication fonctionnelle avec le capteur de liquide de niveau de remplissage de fond et le second le capteur de liquide de niveau de remplissage, la circuiterie de commande étant configurée pour :
générer une indication de second niveau de remplissage indiquant que le ballon est au second niveau de remplissage à un moment où le contenu de liquide du mélange liquide-gaz bouillonnant au second niveau de remplissage dépasse un second seuil de niveau de remplissage ;
générer une indication du niveau de remplissage de fond indiquant que le ballon est au niveau de remplissage de fond à un moment où :
le contenu de liquide du mélange liquide-gaz bouillonnant au niveau de remplissage de fond dépasse un seuil de niveau de remplissage de fond ; et
le contenu de liquide du mélange liquide-gaz bouillonnant au second niveau de remplissage dépasse un seuil supplémentaire, le seuil supplémentaire étant différent du second seuil de niveau de remplissage ; et
générer une erreur de ballon vide à un moment où aucune indication de niveau de remplissage n'est générée.

11. Dispositif selon la revendication 10 ou toute autre revendication, dans lequel le canal d'injection de gaz comporte :
une conduite d'injection de gaz ; et
un réservoir accouplé à la conduite d'injection de gaz, le réservoir étant accouplé au ballon et positionné en dessous de celui-ci pour forcer le mélange liquide-gaz bouillonnant vers le haut dans le ballon, et/ou dans lequel le ballon inclut un ballon d'humidification pour humidifier un gaz du mélange liquide-gaz bouillonnant par exposition au contenu liquide, par exemple dans lequel le contenu liquide comporte un contenu d'eau, par exemple dans lequel le dispositif est inclus dans un système d'humidification de gaz d'un incubateur, dans lequel :
facultativement, le système d'humidification de gaz comporte de multiples ballons d'humidification parallèles incluant le ballon d'humidification ;
facultativement, les multiples ballons d'humidification parallèles diffèrent par leur configuration et un ou plusieurs paramètres opérationnels ;
facultativement, l'incubateur est inclus dans un système de technologie de procréation assistée (ART).

12. Procédé comportant :
l'obtention d'une première lecture mesurée à partir d'un premier capteur de liquide, la première lecture mesurée indiquant une première quantité de liquide présente à un premier niveau de remplissage d'un ballon ;
l'obtention d'une seconde lecture mesurée à partir d'un second capteur de liquide, la seconde lecture mesurée indiquant une seconde quantité de liquide présente à un second niveau de remplissage d'un ballon, le second capteur de liquide correspond au second capteur de remplissage de niveau pour lequel le premier capteur de liquide est positionné en dessous ; avec une circuiterie de commande (108) en communication fonctionnelle avec les premier et second capteurs de liquide, **caractérisé en ce que** la circuiterie de commande est configurée pour déterminer qu'une ou plusieurs conditions de seuil unique sont remplies à un moment où la seconde lecture mesurée dépasse un second seuil de niveau de remplissage ;
la détermination du fait qu'une condition de seuils multiples est remplie à un moment où :
la première lecture mesurée dépasse un premier seuil de niveau de remplissage ; et
la seconde lecture mesurée dépasse un seuil supplémentaire, le seuil supplémentaire étant différent du second seuil de niveau de remplissage ;
la génération d'une indication de niveau de remplissage élevé indiquant que le ballon est rempli au moins jusqu'au second niveau de remplissage à un moment où la ou les conditions de seuil unique sont remplies ; et
la génération d'une indication de premier niveau de remplissage indiquant que le ballon est rempli jusqu'au premier niveau de remplissage à un moment où la condition de seuils multiples est remplie.

13. Procédé selon la revendication 12 pour un dispositif selon la revendication 10 ou 11, comportant en outre l'un parmi :
(a) à un moment où la ou les conditions de seuil unique sont remplies et où la condition de seuils multiples est remplie, l'omission de l'indication de premier niveau de remplissage (b) le fait de provoquer des turbulences dans le liquide présent dans le ballon en forçant le gaz vers le haut à travers le fond du ballon (c) l'interrogation des capteurs de liquide à un intervalle ; et
la génération d'une indication de niveau de remplissage ou d'une erreur de ballon vide à chaque intervalle.

14. Procédé selon la revendication 13 ou l'une quelconque des autres revendications, dans lequel :
l'obtention de la première lecture mesurée et de la seconde lecture mesurée comporte l'une parmi :
(a) la soustraction d'une première valeur étalonnée d'une première lecture brute du premier capteur de liquide pour obtenir la première lecture mesurée ; et
la soustraction d'une seconde valeur étalonnée d'une seconde lecture brute du second capteur de liquide pour obtenir la première lecture mesurée ; et
(b) l'application d'un premier ajustement dépendant de la température à une première lecture brute du premier capteur de liquide pour obtenir la première lecture mesurée ; et
l'application d'un second ajustement dépendant de la température à une seconde lecture brute du second capteur de liquide pour obtenir la seconde lecture mesurée.

15. Procédé comportant :
la génération d'une indication de niveau de remplissage élevé indiquant qu'un ballon est à un niveau de remplissage élevé à un moment où une lecture mesurée d'un capteur de liquide élevé dépasse un seuil de niveau de remplissage élevé, le capteur de liquide élevé étant positionné pour correspondre à un niveau de remplissage élevé d'un ballon, pour lequel un capteur de liquide de fond est positionné en dessous ; avec une circuiterie de commande (108) en communication fonctionnelle avec le capteur de liquide de fond et le capteur de liquide élevé, **caractérisé en ce que**, la circuiterie de commande est configurée pour générer une indication de niveau de remplissage de fond indiquant que le ballon est au niveau de remplissage de fond à un moment où :
une lecture mesurée du capteur de liquide de fond dépasse un seuil de niveau de remplissage de fond ; le capteur de liquide de fond étant positionné pour correspondre à un niveau de remplissage de fond d'un ballon ; et
la lecture mesurée du capteur de liquide élevé dépasse un seuil supplémentaire, le seuil supplémentaire étant différent du seuil de niveau de remplissage élevé ;
et la génération d'une erreur de ballon vide à un moment où aucune indication de niveau de remplissage n'est générée.
